# EUROPÄISCHE PATENTSCHRIFT

(11) **EP 1 000 004 B1**
(45) Veröffentlichungstag und Bekanntmachung des Hinweises auf die Patenterteilung: **02.10.2002**
(21) Anmeldenummer: 98939566.0
(22) Anmeldetag: 30.06.1998
(51) Int. Cl.: C07C 39/00

(54) **VERFAHREN ZUR HERSTELLUNG VON TETRABUTYLAMMONIUMPHENOLAT-DIPHENOLADDUKT**
METHOD FOR PRODUCING TETRABUTYL AMMONIUM PHENOLATE DIPHENOL ADDUCT
PROCEDE POUR LA PREPARATION D'UN PRODUIT D'ADDITION DE DIPHENOLE DE TETRABUTYLAMMONIUMPHENATE

(30) Priorität: 11.07.1997 DE 19730022
(43) Veröffentlichungstag der Anmeldung: 17.05.2000
(73) Patentinhaber: BAYER AG, 51368 Leverkusen (DE)
(72) Erfinder: HESSE, Carsten, D-47800 Krefeld (DE); JANSEN, Ursula, D-47799 Krefeld (DE); RECHNER, Johann, D-47906 Kempen (DE)
(86) Internationale Anmeldenummer: EP9803987
(87) Internationale Veröffentlichungsnummer: WO99002474

(56) Entgegenhaltungen:
- EP-A- 0 362 854
- HANSON, A. W. ET AL: "Complexes of aromatic hydroxy compounds with ammonium salts and amines. Novel hydrogen-bonding networks" TETRAHEDRON LETT. (1982), 23(6), 607-10 , XP002089925 in der Anmeldung erwähnt
- MAGONSKI J. ET AL.: "Dissociation constants of substituted phenols and homoconjugation constants of the corresponding phenol-phenolate systems in acetonitrile" J. CHEM. SOC. FARADAY TRANS., Bd. 89, Nr. 1, 1993, Seiten 119-122, XP002089926 in der Anmeldung erwähnt

## Beschreibung

Die Erfindung betrifft ein Verfahren zur Herstellung und Reinigung von Tetrabutylammoniumphenolat-Diphenoladdukt durch Umsetzung von Alkaliphenolat mit Tetrabutylammoniumsalzen in wäßriger Phase und anschließende Fällung des Adduktes durch Zugabe von Phenol.

Tetraalkylammoniumphenolate sind bereits verschiedentlich bekannt geworden. So offenbaren J. Am. Chem. Soc. 103 (1983) 475 und Inorg. Chem. 24 (1985) 3465 die Herstellung von Tetraethylammoniumphenolat, aus DE-OS 22 03 448 ist die Darstellung von Tetrabutylammoniumphenolat bekannt, EP-A 244 799 lehrt die Herstellung eines Tetraalkylammoniumphenolate enthaltenden Elektrolyten

Auch verschiedene Phenoladdukte von Tetraalkylammoniumphenolaten sind bereits bekannt geworden J. Chem. Soc. Faraday Trans. 89 (1993) 119 offenbart die Herstellung von (Mono-)Phenoladdukten verschiedener Tetrabutylammoniumphenolate und aus EP-A 362 854 geht die Herstellung des Di(p-tert -butylphenol)-Addukts von Tetrabutylammonium(p-tert.-butylphenolat) hervor.

Tetrahedron Lett. 3 (1982) 607 offenbart einen Weg zur Herstellung von Tctrabutylammoniumphenolat-Diphenoladdukt durch Umsetzung von Tetrabutylammoniumhydroxid mit Phenol in wäßriger Losung. Für eine industrielle Produktion ist der Einsatz des teuren Tetrabutylammoniumhydroxids jedoch unbefriedigend. Es wurde daher ein kostengünstiges Verfahren gesucht, das sich im technischen Maßstab durchführen läßt und zu einem Produkt mit einem niedrigen Gehalt an Alkaliionen führt.

Gegenstand der Erfindung ist ein Verfahren zur Herstellung von Tetrabutylammoniumphenolat-Diphenoladdukt N(C₄H₉)₄OC₆H₅*2 C₆H₅OH, bei dem man in einem ersten Schritt Alkaliphenolat mit stöchiometrischen Mengen Tetrabutylammoniumsalz in wäßriger Phase umsetzt und in einem zweiten Schritt zur Reaktionsmischung zwei Äquivalente Phenol zusetzt und das Addukt ausfällt.

In einer bevorzugten Ausführungsform wird dann in einem dritten Schritt der gebildete Niederschlag abgetrennt und mit Wasser gewaschen, bis im Niederschlag ein Natriumgehalt < 1500 ppm erreicht ist.

In einer anderen bevorzugten Ausführungsform wird in einem dritten Schritt die Reaktionsmischung mit einem organischen Lösungsmittel extrahiert, die organische Phase abgetrennt und vom Losungsmittel befreit.

Die Alkaliphenolate werden in wäßriger Phase mit Tetrabutylammoniumsalzen umgesetzt. Bevorzugt handelt es sich bei den Tetrabutylammoniumsalzen um Salze einwertiger Kationen wie Fluorid, Chlorid, Bromid, Iodid, Tetratluoroborat, Perchlorat, Hexafluorophosphat. Aus Gründen der Wirtschaftlichkeit wird besonders bevorzugt Tetrabutylammoniumbromid eingesetzt.

Zweckmäßigerweise werden Alkalisalz und Tetrabutylammoniumsalz in aquimolaren Mengen umgesetzt. Es kann natürlich auch eine der Komponenten im Überschuß eingesetzt werden. Dies verringert jedoch die Ausbeute und verschlechtert die Wirtschaftlichkeit des Verfahrens.

Bei der Durchfuhrung des erfindungsgemäßen Verfahrens wird man bevorzugt zunächst waßrige Lösungen des Alkaliphenolats und des Tetrabutylammoniumsalzes herstellen und diese dann vereinigen In der Regel wird die Salzkonzentration der Ausgangslösungen etwa 0,6 bis 1,3 mol/l betragen. Es ist aber auch möglich, eine Komponente in Wasser zu lösen und die zweite dann in fester Form zuzusetzen. Bevorzugt wird die Reaktionslosung während der Umsetzung gekühlt, so daß die Temperatur der Lösung Raumtemperatur nicht wesentlich überschreitet.

Im zweiten Schritt des Verfahrens werden der Reaktionsmischung zwei Äquivalente Phenol zugesetzt. Auch hier kann natürlich von der exakten Stöchiometrie abgewichen werden, dies wirkt sich jedoch nachteilig auf die Ausbeute oder die Reinheit des Produkts aus. Bevorzugt wird das Phenol der Reaktionsmischung langsam zugetropft, bevorzugt innerhalb von etwa ein bis zwei Stunden, wobei darauf zu achten ist, daß Reaktionsmischung und Phenol gut vermischt werden. Aus der Reaktionsmischung scheidet sich das komplexe Salz N(C₄H₉)₄OC₆H₅*2 C₆H₅OH ab. Zur Vervollständigung der Reaktion wird bevorzugt noch etwa 60 bis 120 Minuten lang nachgerührt

In einer bevorzugten Ausrührungsform wird dann der gebildete Niederschlag abgetrennt. Dies kann durch die üblichen. dem Fachmann bekannten Methoden geschehen, z.B. durch Filtrieren, Sedimentieren oder Zentrifugieren. Der Niederschlag wird dann mit Wasser gewaschen, bis im Niederschlag ein Natriumgehalt < 1500 ppm, bevorzugt < 750 ppm, besonders bevorzugt < 500 ppm erreicht ist. Damit wird ein Einsatz des Produkts auch in Prozessen möglich, die empfindlich auf die Anwesenheit von Alkali-Ionen reagieren Zweckmäßigerweise wird das erhaltene Produkt anschließend getrocknet. Bevorzugt wird bei der Trocknung eine Temperatur unterhalb des Schmelzpunktes der Verbindung gewählt.

In einer anderen bevorzugten Ausführungsform wird in einem dritten Schritt die Reaktionsmischung mit einem organischen Lösungsmittel extrahiert, die organische Phase abgetrennt und vom Lösungsmittel befreit. Als Extraktionsmittel werden bevorzugt Halogenkohlenwasserstoffe verwendet, besonders bevorzugt Methylenchlorid. Nach der Abtrennung der organischen Phase wird diese bevorzugt mit Wasser gewaschen, um Reste wasserlöslicher Verunreinigungen zu entfernen Anschließend wird in dem Fachmann bekannter Weise die organische Phase von Lösungsmittel befreit, z.B. durch Destillation unter vermindertem Druck Nach diesem Verfahren erhält man ein Produkt mit einem besonders niedrigen Natrium-Gehalt. Dieser liegt in der Regel unterhalb der Nachweisgrenze, d.h. bei Werten 1ppm.

Das nach dem erfindungsgemäßen Verfahren hergestellte Diphenoladdukt des Tetrabutylammoniumphenolats eignet sich insbesodere als Bestandteil von Katalysatorsystemen, wie sie beispielsweise bei der Produktion von Phenolharzen zum Einsatz kommen.

### Beispiele

### Beispiel 1

In einem 6000 ml-Dreihalskolben mit eingebautem Strombrecher, KPG-Rührer, beheizbarem Tropftrichter und Rückflußkühler wurden 510,6 g (3,0 mol) Natriumphenolat-Trihydrat in 1800 ml destilliertem Wasser gelöst (Lösung A). In einem 3-1- Becherglas wurden 967,20 g (3,0 mol) Tetrabutylammoniumbromid in 1800 ml destilliertem Wasser gelöst (Lösung B)

Unter Kühlung wurde anschließend Lösung B zu Lösung A gegeben. Anschließend wurden unter heftigem Rühren 535,8 g (5,7 mol) Phenol während 120 min zugetropft und 120 min nachgerührt. Der gebildete Niederschlag wurde abgesaugt und mit 30 l destilliertem Wasser gewaschen. Der Filterruckstand wurde im Vakuumtrockenschrank getrocknet.
Ausbeute: 1147 g (73% der Theorie)
Na-Gehalt des getrockneten Feststoffes, 570 ppm

### Beispiel 2

Beispiel 1 wurde wiederholt. Es wurde lediglich Natriumphenolat in situ durch Lösen von 120,0 g (3,0 mol) NaOH in 1800 ml destilliertem Wasser und Zugabe von 282,3 g (3,0 mol) geschmolzenem Phenol unter Kühlung hergestellt.
Ausbeute: 1028 g (65% der Theorie)
Na-Gehalt des getrockneten Feststoffes: 430 ppm

### Beispiel 3

In einem 500 ml-Dreihalskolben mit eingebautem Strombrecher, KPG-Rührer, beheizbarem Tropftrichter und Rückflußkühler wurden 113,5 g (0,18 mol) einer 40%igen Tetrabutylammoniumhydroxidlosung mit 72 ml destilliertem Wasser verdünnt. Anschließend wurden 49,4 g (0,53 mol) Phenol innerhalb von 60 min zugetropft und 60 min nachgerührt. Der Niederschlag wurde abgesaugt, mit wenig Wasser gewaschen und anschließend im Vakuumtrockenschrank bis zur Gewichtskonstanz getrocknet.
Ausbeute 71 g (77% der Theorie)
Na-Gehalt des getrockneten Feststoffes: ca. 3 ppm

### Beispiel 4

In einem 500 ml-Dreihalskolben mit eingebautem Strombrecher, KPG-Ruhrer, beheizbarem Tropftrichter und Rückflußkühler wurden 29,8 g (0,18 mol) Natriumphenolat in 80 ml destilliertem Wasser gelöst (Lösung A) In einem 250 ml-Becherglas wurden 48,6 g (0,18 mol) Tetrabutylammoniumchlorid in 200 ml destilliertem Wasser gelöst (Lösung B).

Lösung B wurde unter Kühlung zu Lösung A gegeben. Unter heftigem Rühren wurden anschließend 32,9 g (0,35 mol) Phenol innerhalb von 60 min zugetropft, nach Beendigung wurde 60 min nachgerührt. Der gebildete Niederschlag wurde abgesaugt, mit Wasser gewaschen und anschließend im Vakuumtrockenschrank getrocknet.
Ausbeute: 66,2 g (72% der Theorie)
Na-Gehalt des getrockneten Feststoffes: 250 ppm

### Beispiel 5

In einem 2 l-Dreihalskolben mit eingebautem Strombrecher, KPG-Rührer, beheizbarem Tropftrichter und Rücktlußkühler wurde durch Lösen von 40,0 g (1,0 mol) Natriumhydroxid in 500 ml Wasser und Zugabe von 94,1 g (1,0 mol) Phenol 1 mol Natriumphenolat in 500 ml destilliertem Wasser hergestellt (Lösung A).

In einem 2 l-Becherglas wurden 322,4 g (1,0 mol) Tetrabutylammoniumbromid in 700 ml destilliertem Wasser gelöst (Lösung B)

Losung B wurde zu Lösung A gegeben. Unter heftigem Rühren wurden während 60 min 188,2 g (2 mol) Phenol zugetropft. Die entstehende Suspension wurde 60 min nachgeruhrt. Anschließend wurde dreimal mit 200 ml Methylenchlorid extrahiert. Die vereinigten organischen Phasen wurden einmal mit 150 ml Wasser gewaschen, am Rotationsverdampfer eingeengt und schließlich mit Hilfe einer Ölpumpe getrocknet. Es entstand ein fester Kristallkuchen.
Ausbeute: 514,0 g (98% der Theorie)
Na-Gehalt des Feststoffes: < 1 ppm

Der Bromidgehalt lag bei 3,8 Gew -%, was berechnet als Tetrabutylammoniumbromid einem Gehalt von 15,3 Gew.-% entspricht.

### Beispiel 6

In einem 100 l Rührwerksbehalter aus Email mit Kühlwasseranschluß, Destillationsaufbau, Vakuum-Stickstoffanschluß und beheizbarem Dosiergefaß wurden 19.7 kg (61,6 mol) Tetrabutylammoniumbromid mit 30.5 l VE-Wasser gelost und zur Zwischenlagerung in ein mit Valenthene® ausgekleidetes Faß abgefüllt (Lösung A).

In einer analogen Apparatur wurden in 15,3 kg VE-Wasser 1,22 kg (30.5 mol) NaOH gelöst und die Losung mit 2,83 kg (30,1 mol) geschmolzenem Phenol versetzt (Lösung B).

Anschließend wurden 25 kg Lösung A (= 30,5 mol Tetrabutylammoniumbromid) zugepumpt und gut vermischt. Aus dem beheizbaren Tropftrichter wurden dann 5,6 kg (59,5 mol) Phenol unter guter Durchmischung bei Raumtemperatur innerhalb von 140 min zugetropft. Es wurde 90 min nachgerührt und anschließend uber eine große Nutsche, die mit einem Dralontuch bespannt war, abfiltriert. Nachdem mit Wasser gewaschen worden war, wurde der Ruckstand auf Blechen in einem Vakuumtrockenschrank bei 25°C bis zur Gewichtskonstanz getrocknet.
Ausbeute: 10,1 kg (63% der Theorie)
Na-Gehalt: 150 ppm; Bromid-Gehalt: 0.12 Gew.-%; Wassergehalt: 400 ppm

### Beispiel 7 (Vergleichsbeispiel)

In einem 250 ml-Becherglas wurden 8, 1 g (0,09 mol) Phenol in 100 ml Wasser gelöst. In einem 100 ml-Becherglas wurden 55 ml einer 40%igen Tetrabutylammoniumhydroxid-Lösung (= 0,085 mol) mit 25 ml destilliertem Wasser verdunnt Beide Lösungen wurden in einem Schütteltrichter vereinigt, ca. 5 min gut vermischt und die Phasen getrennt. Die organische Phase wurde über MgSO₄ getrocknet und nach Filtration eingedampft. Der zähflüssige Rückstand wurde in 11 ml Ethylacetat aufgenommen. Es gelang jedoch nicht, aus der Lösung ein kristallines Produkt zu isolieren. Auch die Zugabe von Impfkristallen bei ca. 0 °C führte nicht zur Kristallisation.

## Patentansprüche

1. Verfahren zur Herstellung von Tetrabutylammoniumphenolat-Diphenoladdukt, bei dem man in einem ersten Schritt Alkaliphenolat mit stöchiometrischen Mengen Tetrabutylammoniumsalz in wäßriger Phase umsetzt und in einem zweiten Schritt zur Reaktionsmischung zwei Äquivalente Phenol zusetzt und das Addukt ausfällt.

2. Verfahren gemäß Anspruch 1, bei dem man in einem dritten Schritt den gebildeten Niederschlag abtrennt und mit Wasser wäscht, bis im Niederschlag ein Natriumgehalt < 1500 ppm erreicht ist.

3. Verfahren gemäß Anspruch 1, bei dem man in einem dritten Schritt die Reaktionsmischung mit einem organischen Lösungsmittel extrahiert, die organische Phase abtrennt und vom Lösungsmittel befreit.

## Claims

1. Process for the production of tetrabutylammonium phenolate diphenol adduct, wherein, in a first step, alkali phenolate is caused to react with stoichiometric quantities of tetrabutylammonium salt in the aqueous phase and, in a second step, two equivalents of phenol are added to the reaction mixture and the adduct is precipitated out.

2. Process according to Claim 1, wherein, in a third step, the precipitate that is formed is separated and washed with water until a sodium content of < 1,500 ppm in the precipitate is attained.

3. Process according to Claim 1, wherein, in a third step, the reaction mixture is extracted with an organic solvent, the organic phase is separated and freed from solvent.

## Revendications

1. Procédé pour la préparation d'un produit d'addition de phénolate de tétrabutylammonium-diphénol, **caractérisé en ce que** l'on fait réagir dans une première étape un phénolate d'alcali avec des quantités stoechiométriques de sel de tétrabutylammonium en phase aqueuse et **en ce que** l'on ajoute dans une seconde étape au mélange réactionnel deux équivalents de phénol et on fait précipiter le produit d'addition.

2. Procédé selon la revendication 1, dans lequel on sépare dans une troisième étape le précipité formé et on le lave avec de l'eau jusqu'à ce que l'on atteigne une teneur en sodium < 1500 ppm dans le précipité.

3. Procédé selon la revendication 1, dans lequel on extrait dans une troisième étape le mélange réactionnel avec un solvant organique, on sépare la phase organique et on la libère du solvant.
